# EUROPEAN PATENT SPECIFICATION

(11) **EP 3 250 188 B1**
(45) Date of publication and mention of the grant of the patent: **14.09.2022**
(21) Application number: 16703060.0
(22) Date of filing: 27.01.2016
(51) Int. Cl.: A61K 9/14, A61K 9/19, A61K 9/48, A61K 31/5377

(54) **PHARMACEUTICAL COMPOSITION COMPRISING APREPITANT AND METHOD FOR THE PREPARATION THEREOF**
PHARMAZEUTISCHE ZUSAMMENSETZUNG MIT APREPITANT UND VERFAHREN ZUR HERSTELLUNG DAVON
COMPOSITION PHARMACEUTIQUE COMPRENANT DE L'APRÉPITANT ET SON PROCÉDÉ DE PRÉPARATION

(30) Priority: 30.01.2015 GR 20150100035; 27.10.2015 GR 20150100471
(43) Date of publication of application: 06.12.2017
(73) Proprietor: Pharmathen S.A., Pallini Attikis 15351 (GR)
(72) Inventor: KARAVAS, Evangelos, 153 51 Pallini Attikis (GR); KOUTRIS, Efthymios, 153 51 Pallini Attikis (GR); SAMARA, Vasiliki, 153 51 Pallini Attikis (GR); KOUTRI, Ioanna, 153 51 Pallini Attikis (GR); KALASKANI, Anastasia, 153 51 Pallini Attikis (GR); KIZIRIDI, Christina, 153 51 Pallini Attikis (GR); ABATZIS, Morfis, 153 51 Attikis (GR); BIKIARIS, Dimitrios, 153 51 Pallini Attikis (GR)
(86) International application number: PCT/EP2016/000136
(87) International publication number: WO 2016/120013

(56) References cited:
- EP-A1- 2 813 212
- EP-A1- 2 893 919
- WO-A1-2008/110534
- WO-A1-2008/110543
- WO-A2-2007/147160
- ALFRED E TIEFENBACHER (GMBH & CO KG) AND AET LABORATORIES PVT LTD: "Improved Aprepitant Formulations Containing Soluplus and NaCl", RESEARCH DISCLOSURE, KENNETH MASON PUBLICATIONS, HAMPSHIRE, UK, GB, vol. 608, no. 33, 1 December 2014 (2014-12-01), page 1, XP007143650, ISSN: 0374-4353 [retrieved on 2014-11-18]
- Chandrasekhara Rao ET AL: "Dissolution Enhancement of Poorly Soluble Drug Aprepitant by Hot Melt Extrusion Method Using Hydrophilic Polymer: A Solid Dispersion Technique", Research Journal of Pharmaceutical, Biological and Chemical Sciences, 1 January 2014 (2014-01-01), XP055697411, Retrieved from the Internet: URL:http://www.rjpbcs.com/pdf/2014_5(3)/ 1 53!.pdf [retrieved on 2020-05-20]
- Ritesh A. Fule ET AL: "Artemether-Soluplus Hot-Melt Extrudate Solid Dispersion Systems for Solubility and Dissolution Rate Enhancement with Amorphous State Characteristics", Journal of Pharmaceutics, vol. 2013, 1 January 2013 (2013-01-01), pages 1-15, XP055698366, ISSN: 2090-9918, DOI: 10.1155/2013/151432
- DATABASE MEDLINE [Online] US NATIONAL LIBRARY OF MEDICINE (NLM), BETHESDA, MD, US; 10 April 2015 (2015-04-10), PUNCOCHOVÁ KATERINA ET AL: "Identifying the mechanisms of drug release from amorphous solid dispersions using MRI and ATR-FTIR spectroscopic imaging.", Database accession no. NLM25686660 & PUNCOCHOVÁ KATERINA ET AL: "Identifying the mechanisms of drug release from amorphous solid dispersions using MRI and ATR-FTIR spectroscopic imaging.", INTERNATIONAL JOURNAL OF PHARMACEUTICS 10 APR 2015, vol. 483, no. 1-2, 10 April 2015 (2015-04-10), pages 256-267, ISSN: 1873-3476
- DATABASE EMBASE [Online] ELSEVIER SCIENCE PUBLISHERS, AMSTERDAM, NL; May 2014 (2014-05), CHANDRASEKHARA RAO B ET AL: "Dissolution enhancement of poorly soluble drug aprepitant by hot melt extrusion method using hydrophilic polymer: A solid dispersion technique", Database accession no. EMB-2014351113 & CHANDRASEKHARA RAO B ET AL: "Dissolution enhancement of poorly soluble drug aprepitant by hot melt extrusion method using hydrophilic polymer: A solid dispersion technique", RESEARCH JOURNAL OF PHARMACEUTICAL, BIOLOGICAL AND CHEMICAL SCIENCES MAY/JUNE 2 RESEARCH JOURNAL OF PHARMACEUTICAL, BIOLOGICAL AND CHEMICAL SCIENCES IND, vol. 5, no. 3, May 2014 (2014-05), pages 1469-1485, ISSN: 0975-8585
- "Improved Aprepitant Formulations", RESEARCH DISCLOSURE, KENNETH MASON PUBLICATIONS, HAMPSHIRE, UK, GB, vol. 590, no. 79, 1 June 2013 (2013-06-01) , page 1, XP007142273, ISSN: 0374-4353

## Description

### TECHNICAL FIELD OF THE INVENTION

The present invention relates to an immediate release oral dosage composition of Aprepitant formulated as amorphous solid dispersion and a method for the preparation thereof.

### BACKROUND OF THE INVENTION

Substance P (SP) is a peptide composed of a chain of 11 amino acid residues and member of the tachykinin neuropeptide family. It is a neuropeptide, acting both as a neurotransmitter and as a neuromodulator. It is an important element in pain perception since it is believed to be related to the transmission of pain information into the central nervous system. SP receptor antagonists have important therapeutic applications in the treatment of a variety of stress-related illnesses, in addition to their potential as analgesics.

Aprepitant is an antiemetic chemical compound that belongs to the substance P antagonists (SPA) class of drugs. It mediates its effect by blocking the neurokinin 1 (NK1) receptor. It is a white to off-white color, non-hygroscopic crystalline powder that is practically insoluble in water and sparingly soluble in ethanol, isopropyl acetate while slightly soluble in acetonitrile.

The very low solubility in water is the major problem in the process of producing a pharmaceutically acceptable composition of this active ingredient. A number of solubility enhancement methods are reported in literature and are applied in pharmaceutical industry. Particle size reduction, pH modification in case of pH dependent solubility, use of surfactants and/or solubilizers, complexation with cyclodextrins and lipid based formulations are some of the commonly used methods. European patent 1455765 utilizes the particle size reduction technic were nanoparticulates of average particle size of less than one micron are formulated into a pharmaceutical composition.

WO 2007/112457 discloses compositions of Aprepitant polymorph with a cyclodextrin. EP 2 813 212 A1 discloses an amorphous solid dispersion of Aprepitant in polyvinyl caprolactam - polyvinyl acetate - polyethylene glycol graft copolymer.

EP 2 893 919 A1 discloses an amorphous formulation of Aprepitant in solid solution with polymer Soluplus.

"Improved Aprepitant Formulations", RESEARCH DISCLOSURE, vol. 590, no.79, 01.06.2013, page 1, ISSN: 0374-4353 and ALFRED E TIEFENBACHER (GMBH & CO KG) AND AET LABORATORIES PVT LTD: "Improved Aprepitant Formulations Containing Soluplus and NaCl", RESEARCH DISCLOSURE, vol. 608, no. 33, 01.12.2014, page 1, ISSN: 0374-4353 disclose capsule formulations of aprepitant.

A hot melt extrusion process is used to prepare the aprepitant formulation for filing the capsules. The hot melt extrusion process is stated to be particularly suitable with Soluplus, to improve the solubility of aprepitant. Chandrasekhara Rao ET AL: "Dissolution Enhancement of Poorly Soluble Drug Aprepitant by Hot Melt Extrusion Method Using Hydrophilic Polymer: A Solid Dispersion Technique", Research Journal of Pharmaceutical, Biological and Chemical Sciences, 01.01.2014, URL:http:// www.rjpbcs.com/pdf/2014_5(3)/ 153!.pdf describes a study on the use of hydroxypropyl cellulose (HPC) as a model polymeric carrier for producing solid dispersions of aprepitant using hot melt extrusion.

Ritesh A. Fule ET AL: "Artemether-Soluplus Hot-Melt Extrudate Solid Dispersion Systems for Solubility and Dissolution Rate Enhancement with Amorphous State Characteristics", Journal of Pharmaceutics, vol. 2013, 01.01.2013, pages 1-15, ISSN: 2090-9918, DOI: 10.1155/2013/151432 shows that Soluplus was known for improving solubility of poorly soluble drugs via formation of amorphous solid dispersions using melt mixing processes.

None of the prior art compositions disclose Aprepitant composition as currently claimed in the present invention.

### SUMMARY OF THE INVENTION

This invention relates to novel pharmaceutical compositions of Aprepitant. Such compositions manage to solve the problem of poor solubility of the specific active pharmaceutical ingredient. An alternative formulation easy to manufacture and economic is provided.

Other objects and advantages of the present invention will become apparent to those skilled in the art in view of the following detailed description.

### DETAILED DESCRIPTION OF THE INVENTION

The invention relates to a pharmaceutical composition comprising an amorphous solid dispersion of Aprepitant with polyvinyl caprolactam - polyvinyl acetate - polyethylene glycol graft copolymer, wherein the ratio by weight of Aprepitant to the carrier polyvinyl caprolactam - polyvinyl acetate - polyethylene glycol graft copolymer is from 1:1 to 1:4, which pharmaceutical composition further comprises a diluent, a disintegrant, a glidants and a lubricant and wherein the diluent is lactose.

The invention further relates to a process for the preparation of a pharmaceutical composition of Aprepitant comprising the steps of: - Mixing the granules of an amorphous solid dispersion as defined in claim 1 with a diluent, a disintegrant and a glidant, wherein the diluent is lactose until complete homogeneity is achieved; -Further mixing with a lubricant and filling hard gelatin capsules with the obtained granules.

The compound Aprepitant has the following structure: 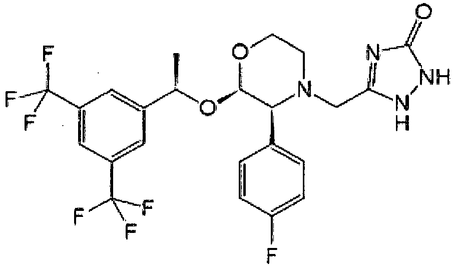 and is a tachykinin receptor antagonist useful for the treatment of disorders mainly relates to central nervous system. It has a molecular formula of C₂₃H₂₁F₇N₄O₃ and a molecular weight of 534.43.

The main object of the present invention was to develop a robust and stable immediate release solid dosage form comprising Aprepitant with enhanced solubility. The solution provided here is the formation of an amorphous solid dispersion. Such method was selected since it is common, simple and reproducible.

For the purposes of the present invention, a pharmaceutical composition comprising an active ingredient is considered to be "stable" if said ingredient degrades less or more slowly than it does on its own and/or in known pharmaceutical compositions.

Generally, amorphous pharmaceutically active ingredients have higher energy than crystalline ones. This leads to better solubility and potentially better bioavailability. For Aprepitant it is reported that the most thermodynamically stable form is crystalline form I, therefore even if an amorphous form was initially selected it would lose its advantages in long term since it would inevitably transform to the more stable crystalline form.

The amorphization process of crystalline Aprepitant through its distribution in an inert carrier was selected in order to maintain the desirable state of the active ingredient. For this purpose both spraying drying and melt extrusion were proven adequate manufacturing processes yet still relatively simple and cost effective.

Applicants have unexpectedly found that an amorphous solid dispersion of Aprepitant with polyvinyl caprolactam - polyvinyl acetate - polyethylene glycol graft copolymer improves significantly the solubility and still provide a stable pharmaceutical composition in long term storage thus avoiding unwanted impurities that would jeopardize the products' effectiveness or the patients' health.

Aprepitant in the present invention may have a size of D₉₀ from 1 to 20 microns as measured with a Malvern Mastersizer, more preferably from 1 to 10 microns and most preferable from 1 to 5 microns.

The carrier used in the amorphous solid dispersion is polyvinyl caprolactam - polyvinyl acetate - polyethylene glycol graft copolymer.

The amount of active pharmaceutical ingredient as a ratio by weight to the carrier used in the present invention is from 1:1 to 1:4, more preferably 1:1.5.

Moreover, the pharmaceutical compositions of the present invention may also contain one or more additional formulation excipient such as diluents, disintegrants, binders, lubricants and glidants, provided that they are compatible with the active ingredient of the composition, so that it does not interfere with it in the composition and in order to increase the stability of the drug and the self-life of the pharmaceutical product.

Diluents may be, for example, dextrates, dextrose, fructose, mannitol, sorbitol, starch, pregelatinized starch, sucrose, xylitol, maltose, maltodextrin, maltitol. Disintegrants may be selected from alginic acid, carbon dioxide, carboxymethylcellulose calcium, carboxymethylcellulose sodium, croscarmelose sodium, guar gum, methylcellulose, polacrilin potassium, poloxamer, sodium alginate. Binders may be, for example, alginic acid, carbomer, ethyl cellulose, gelatin, liquid glucose, guar gum, hydroxyethyl cellulose, povidone, methylcellulose, polydextrose, polyethylene oxide.

Also, at least a lubricant is incorporated into the formulation to prevent the powder from adhering to tablet punches during the compression procedure. Lubricants may be, for example, talc, magnesium stearate, calcium stearate, glyceryl behenate, hydrogenated castor oil, stearic acid, sodium lauryl sulfate.

Glidants are used to promote powder flow by reducing interparticle friction and cohesion. These are used in combination with lubricants as they have no ability to reduce die wall friction. Glidants may be, for example, colloidal silicon dioxide, calcium silicate, calcium phosphate tribasic.

There are also provided methods for preparing the amorphous solid dispersion of Aprepitant of the present invention.

More specifically, a method of manufacturing comprises the steps of:
(a) Dissolving the API and the carrier in ethanol under heating
(b) Spray drying the solution where the solvent is evaporated
(c) Collecting the solid material

Another method for preparing the amorphous solid dispersion comprises the steps of:
(a) API and carrier are added together in a drum under high temperature and continuous mixing
(b) When a homogenous slurry is achieved the mixture is cooled down to room temperature, pulverized and sifted
(c) Collecting the solid material

The present invention also relates to a process for preparing a pharmaceutical formulation of Aprepitant as detailed above.

The following examples illustrate preferred embodiments in accordance with the present invention without limiting the scope of the invention.

### EXAMPLES

### Example 1

### Spray drying

Spray drying experiments were performed in a Buchi B-290 spray drier using as carriers Povidone, Copovidone or polyvinyl caprolactam - polyvinyl acetate - polyethylene glycol graft copolymer.

The ratios of Aprepitant with each carrier applied were 1: 4, 1: 2.5, 1: 1.5 and 1:1. All solid dispersions were prepared by dissolving the API and the polymer in ethanol under heating at about 40°C and then spraying the solution in the spray drier in which the solvent is evaporated and the solid material is collected in a vessel.

The solid dispersions that were obtained were tested through XRD analysis in order to monitor the solid state of the API in the solid dispersion. According to the XRD analysis data, the solid dispersions of Aprepitant with any of the polymers mentioned above which were prepared by the spray drying technique contain the active ingredient in amorphous form since none of the peaks of crystalline Aprepitant is present in the diffractograms of the solid dispersions. In addition, amorphization of Aprepitant is achieved in all the ratios with the polymers from 1:4 to 1:1.

### Solubility and Dissolution studies

The solid dispersions prepared by spray drying were tested for their solubility and dissolution properties in comparison to the crystalline API.

For the solubility study, a quantity of the solid dispersion equivalent to 10.0 mg of Aprepitant was dissolved in a 100ml volumetric flask with dissolution medium of fed state simulated intestinal fluid (FeSSIF) to a concentration of 0.1mg/ml. The solution was magnetically stirred for 30min and before injected in the HPLC it was centrifuged at 4000rpm for 5min. Finally the drug concentration was equated to the drug solubility.

**Table 1: Solubility of solid dispersion prepared according to example 1**

| **Component** | **Solubility in FeSSIF (mg/ml)** |
|---|---|
| Aprepitant API | 0.050 |
| Aprepitant /Povidone 1:4 | 0.097 |
| Aprepitant /Povidone 1:2.5 | 0.099 |
| Aprepitant /Povidone 1:1.5 | 0.072 |
| Aprepitant /Povidone 1:1 | 0.073 |
| Aprepitant /Copovidone 1:4 | 0.099 |
| Aprepitant /Copovidone 1:2.5 | 0.090 |
| Aprepitant /Copovidone 1:1.5 | 0.084 |
| Aprepitant /Copovidone 1:1 | 0.069 |
| Aprepitant / polyvinyl caprolactam - polyvinyl acetate - polyethylene glycol graft copolymer 1:4 | 0.102 |
| Aprepitant / polyvinyl caprolactam - polyvinyl acetate - polyethylene glycol graft copolymer 1:2.5 | 0.101 |
| Aprepitant / polyvinyl caprolactam - polyvinyl acetate - polyethylene glycol graft copolymer 1:1.5 | 0.097 |
| Aprepitant / polyvinyl caprolactam - polyvinyl acetate - polyethylene glycol graft copolymer 1:1 | 0.096 |

The rate and extent of drug dissolution was studied using a USP type II apparatus (paddles) at 50 rpm. The dissolution medium was a FeSSIF with pH 5.0. The release rates of the solid dispersions are compared with that of the pure active ingredient and are presented in table 2 below.

**Table 2: Dissolution results of amorphous solid dispersions for different ratios of API to polymer compared with that of Aprepitant alone.**

| **Time (min)** | **Aprepitant** | **API : Povidone** | | | | **API : Polyvinyl caprolactam-polyvinyl acetate-polyethylene glycol graft copolymer** | | | | **API : Copovidone** | | | |
|---|---|---|---|---|---|---|---|---|---|---|---|---|---|
| | | **1:4** | **1:2.5** | **1:1.5** | **1:1** | **1:4** | **1:2.5** | **1:1.5** | **1:1** | **1:4** | **1:2.5** | **1:1.5** | **1:1** |
| | **% Release** | | | | | | | | | | | | |
| 15 | 17,37 | 77,22 | 69,26 | 67,60 | 64,81 | 86,56 | 75,34 | 75,10 | 77,90 | 80,46 | 76,54 | 71,67 | 69,00 |
| 30 | 38,74 | 80,75 | 70,67 | 71,12 | 66,57 | 90,35 | 81,50 | 78,94 | 78,80 | 85,76 | 82,46 | 79,93 | 71,25 |
| 45 | 45,34 | 81,08 | 70,86 | 73,38 | 69,03 | 95,95 | 86,45 | 83,50 | 81,24 | 89,25 | 90,50 | 81,37 | 75,51 |
| 60 | 47,38 | 83,15 | 74,40 | 74,17 | 69,14 | 97,10 | 90,00 | 88,70 | 87,62 | 89,80 | 91,47 | 86,64 | 79,26 |

According to the results presented above a significant enhancement in dissolution of Aprepitant is achieved through the amorphous solid dispersions which provide an almost instant dissolution. The solid dispersion of Aprepitant to Polyvinyl caprolactam-polyvinyl acetate-polyethylene glycol graft copolymer presented adequate release rate in all the studied ratios from 1:1 to 1:4.

### Finished dosage form

Powders prepared by spray drying were further formulated with other excipients and filled into capsules in order to improve their physical properties like flowability and their dissolution properties were also tested. Without been limited the solid dispersion of Aprepitant with Polyvinyl caprolactam-polyvinyl acetate-polyethylene glycol graft copolymer at the ratio of 1:1.5 was tested.

The spray dried powders were mixed with a diluent such as lactose, mannitol or microcrystalline cellulose, a disintegrant such as crospovidone. Also, colloidal silicon dioxide was used as a glidant and magnesium stearate as lubricant.

**Table 3: Finished dosage forms of solid dispersion of Aprepitant with Polyvinyl caprolactam-polyvinyl acetate-polyethylene glycol graft copolymer at the ratio of 1:1.5**

| **Ingredients** | **Trial1A** | **Trial1B** | **Trial1C** |
|---|---|---|---|
| | % | % | % |
| Solid dispersion Aprepitant/ Polyvinyl caprolactam-polyvinyl acetate-polyethylene glycol graft copolymer 1:1.5 | 80,13 | 80,13 | 80,13 |
| Lactose anhydrous | 12,87 | - | - |
| Mannitol | - | 12,87 | - |
| Microcrystalline cellulose | - | - | 12,87 |
| Crospovidone | 5,00 | 5,00 | 5,00 |
| Colloidal silicon dioxide | 1,00 | 1,00 | 1,00 |
| Magnesium stearate | 1,00 | 1,00 | 1,00 |
| **Total** | 100,00 | 100,00 | 100,00 |

Manufacturing process:
1. The solid dispersion obtained through spray drying process is sifted through 40 mesh sieve.
2. The powder is mixed with the diluent, Crospovidone and Colloidal silicon dioxide.
3. Finally the blend is mixed with magnesium stearate and then filled into capsules.

As presented in table 4 below, the use of flow aid excipients improve the flow properties of the spray dried powder and can thus be formulated in the final dosage form more easily. Also lactose provides the best results out of the tested diluents.

**Table 4: Flow properties of formulations of example 1**

| | **Carr's index** |
|---|---|
| **Solid dispersion Aprepitant/ Polyvinyl caprolactam-polyvinyl acetate-polyethylene glycol graft copolymer 1:1.5** | **36.8%** |
| **Trial 1A** | 23.0% |
| **Trial 1B** | 23.7% |
| **Trial 1C** | 22.0% |

According to the presented data the solubility and the dissolution properties (table 5) of the solid dispersion of Aprepitant/ Polyvinyl caprolactam-polyvinyl acetate-polyethylene glycol graft copolymer were enhanced showing that the presence of other soluble excipients like lactose or mannitol promotes the dissolution of Aprepitant from the solid dispersion.

**Table 5: Dissolution profiles of formulations of example 1**

| **Time (min)** | **Trial 1A % release** | **Trial 1B % release** | **Trial 1C % release** |
|---|---|---|---|
| 15 | 91,33 | 84,84 | 81,55 |
| 30 | 94,29 | 86,50 | 86,31 |
| 45 | 97,41 | 88,67 | 87,25 |
| 60 | 99,65 | 89,50 | 88,55 |

### Stability

In order to study the physical and chemical stability of the solid dispersions prepared by spray drying, samples were stored under normal and accelerated conditions (25°C/60% RH and 40°C/ 75% RH respectively) in powder form as well as in the final dosage form.

Physical stability was studied through XRD analysis in terms of solid state of the active ingredient. According to the results the amorphous state of Aprepitant in the solid dispersions with Povidone, Polyvinyl caprolactam-polyvinyl acetate-polyethylene glycol graft copolymer and Copovidone is maintained after 6 months storage under normal and accelerated conditions.

Also the chemical stability was studied in terms of impurities and the results are presented below.

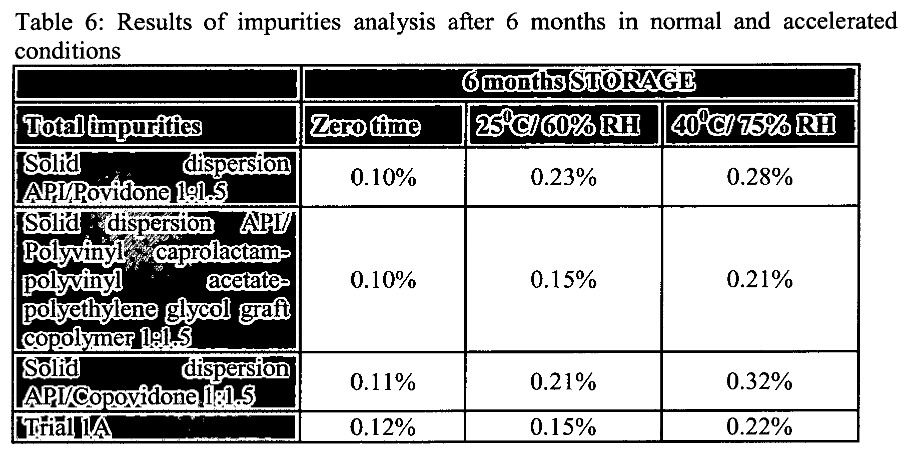

### Example 2

### Melt mixing

Another method applied for the preparation of solid dispersions of Aprepitant was the melt mixing technique according to which Aprepitant is embedded in a carrier/polymer which is melt and upon cooling it is molecularly dispersed in the polymer forming solid dispersion.

Aprepitant has a melting point of 253°C. Such high temperatures are required in order to be miscible with the polymer/carrier in its melt. Povidone has a high glass transition temperature (T_{g}= 167 ⁰C). This T_{g} value indicates that increased temperatures are needed to prepare solid dispersions by melt extrusion. Thus, in order to decrease the T_{g} value of PVP and hence decrease the temperature of extrusion, plasticizers may be used, such as PEG of low molecular weight. Polyvinyl caprolactam-polyvinyl acetate-polyethylene glycol graft copolymer has low glass transition temperature (T_{g} ≈ 70 ⁰C), is easily extrudable and thus there is no need of plasticizer addition in the formulation. It also has good thermal stability, good flowability and low toxicity.

Melt mixing of Aprepitant with Polyvinyl caprolactam-polyvinyl acetate-polyethylene glycol graft copolymer and Povidone in combination with PEG as plasticizer (in ratios 80:20 and 70:30) was performed at ratios 1:4, 1:2.5 and 1:1.5 and in temperatures from 160°C to 200°C. After cooling of the mixtures they were pulverized and sifted through 100mesh sieve. In the following table are presented, the solid dispersions that were prepared, the conditions as well as the solid state of the active ingredient in the solid dispersion.

**Table 7: Solid dispersions prepared by melt mixing**

| **Solid dispersion** | **Temperature** | **Time** | **Solid state (XRD)** |
|---|---|---|---|
| API/Graft copolymer 1:4 | 160°C | 15min | crystalline |
| API/Graft copolymer 1:1.5 | | | crystalline |
| API/Graft copolymer 1:4 | 180°C | 10min | amorphous |
| API/Graft copolymer 1:1.5 | | | crystalline |
| API/Graft copolymer 1:4 | 200°C | 10min | amorphous |
| API/Graft copolymer 1:1.5 | | | amorphous |
| API/(PVP/PEG 80:20) 1:4 | 180°C | 15min | amorphous |
| API/(PVP/PEG 80:20) 1:1.5 | | | crystalline |
| API/(PVP/PEG 80:20) 1:4 | 200°C | 15min | amorphous |
| API/(PVP/PEG 80:20) 1:1.5 | | | amorphous |
| API/(PVP/PEG 70:30) 1:4 | 180°C | 15min | amorphous |
| API/(PVP/PEG 70:30) 1:1.5 | | | crystalline |
| API/(PVP/PEG 70:30) 1:4 | 200°C | 15min | amorphous |
| API/(PVP/PEG 70:30) 1:1.5 | | | crystalline |

According to the X-ray diffractograms, combination of the proper ratio with the polymer and process temperature above 180⁰C results in amorphous solid dispersion of Aprepitant whereas temperature lower than 180⁰C is not efficient to convert the API to amorphous due to its high melting point.

### Solubility and Dissolution

The solid dispersions were tested for their solubility and the results are presented in the following table.

**Table 8: Solubilities of solid dispersions prepared by melt mixing**

| **Component** | **Solubility in FeSSIF (mg/ml)** |
|---|---|
| Aprepitant API | 0.050 |
| API/ Graft copolymer 1:4 160°C | 0.056 |
| API/ Graft copolymer 1:1.5 160⁰C | 0.044 |
| API/ Graft copolymer 1:4 180⁰C | 0.090 |
| API/ Graft copolymer 1:1.5 180⁰C | 0.081 |
| API/ Graft copolymer 1:4 200⁰C | 0.097 |
| API/ Graft copolymer 1:1.5 200⁰C | 0.090 |
| API/(PVP/PEG 80:20) 1:4 180°C | 0.094 |
| API/(PVP/PEG 80:20) 1:1.5 180⁰C | 0.058 |
| API/(PVP/PEG 80:20) 1:4 200⁰C | 0.101 |
| API/(PVP/PEG 80:20) 1:1.5 200⁰C | 0.086 |
| API/(PVP/PEG 70:30) 1:4 180⁰C | 0.094 |
| API/(PVP/PEG 70:30) 1:1.5 180⁰C | 0.056 |
| API/(PVP/PEG 70:30) 1:4 200⁰C | 0.092 |
| API/(PVP/PEG 70:30) 1:1.5 200⁰C | 0.059 |

The solubility results confirm the improvement when the Aprepitant is in the form of a solid dispersion with specific polymer to API ratio & melt mixing temperatures.

The dissolution rate of the solid dispersions that present the best solubility was studied in USP II apparatus, 50rpm, FeSSIF medium.

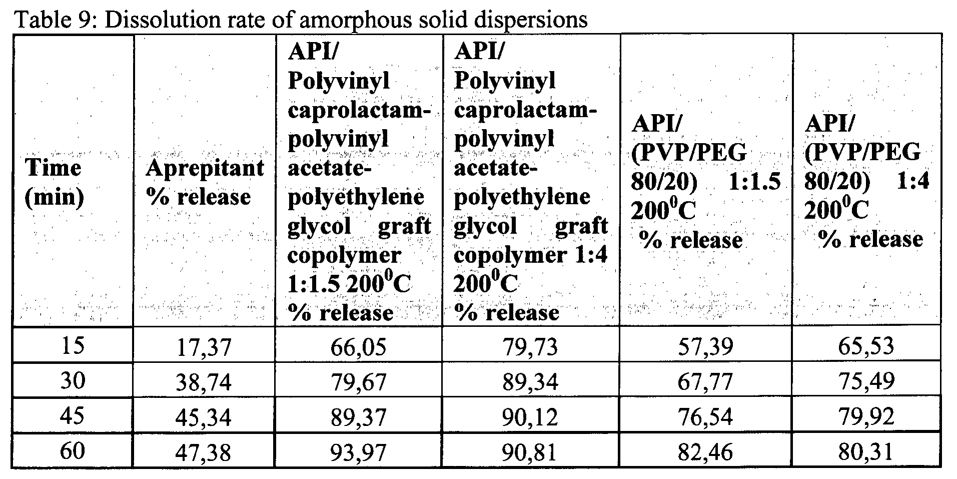

The results indicate that solid dispersions of Aprepitant with Polyvinyl caprolactam-polyvinyl acetate-polyethylene glycol graft copolymer or PVP /PEG prepared by melt mixing exhibit much higher solubility than pure Aprepitant.

### Finished dosage form

The solid dispersion of Aprepitant with Polyvinyl caprolactam-polyvinyl acetate-polyethylene glycol graft copolymer 1:1.5 prepared at 200⁰C was further mixed with other excipients and filled in a capsule (trial 2) and was analyzed for its dissolution properties.

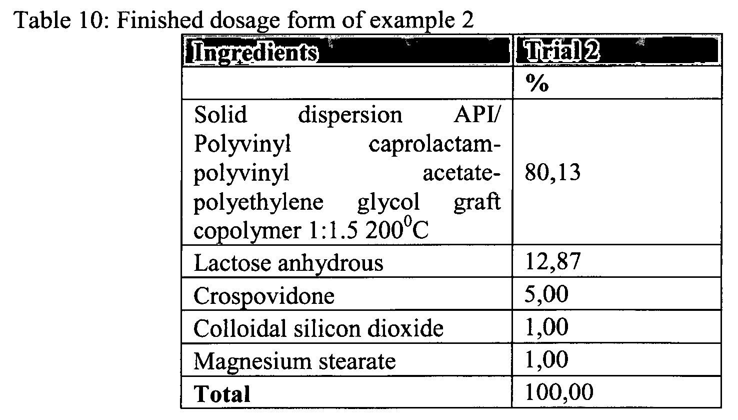

Manufacturing process:
1. The solid dispersion obtained through melt mixing process is sifted through 100 mesh sieve.
2. The powder is mixed with Lactose anhydrous, Crospovidone and Colloidal silicon dioxide.
3. Finally the blend is mixed with magnesium stearate and then filled into capsules

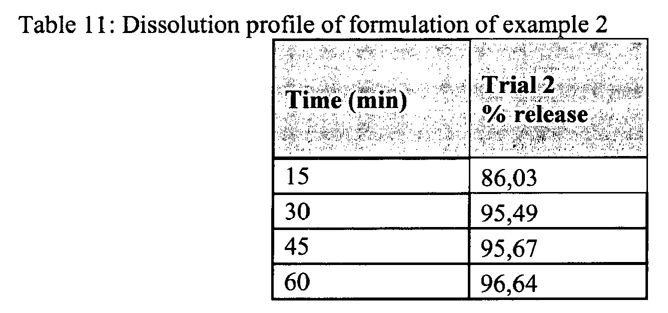

### Stability

The physical and chemical stability of the solid dispersions prepared by melt mixing in powder form and in final dosage form was also studied with samples stored under normal and accelerated conditions (25°C/60% RH and 40°C/75% RH).

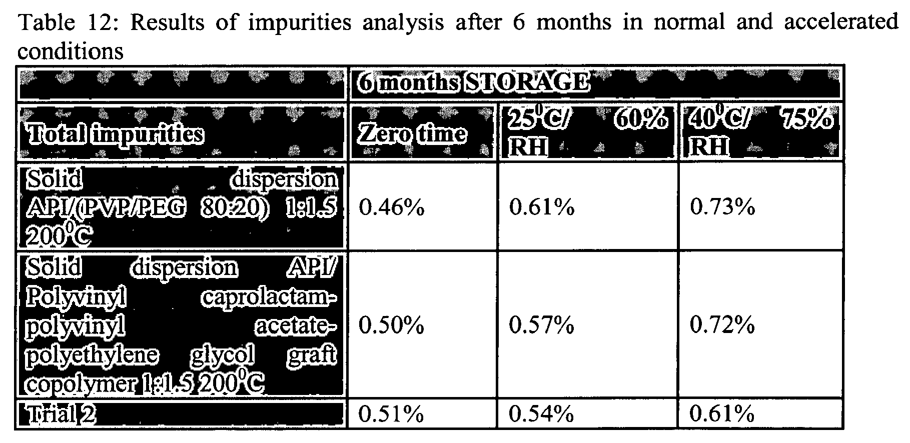

According to the stability data, the melt mixing process results in physical and chemical stability of Aprepitant in its amorphous solid dispersion after 6 months storage under normal and accelerated conditions.

While the present invention has been described with respect to the particular embodiments, it will be apparent to those skilled in the art that various changes and modifications may be made in the invention without departing from the scope thereof, as defined in the appended claims.

## Claims

1. A pharmaceutical composition comprising an amorphous solid dispersion of Aprepitant with polyvinyl caprolactam - polyvinyl acetate - polyethylene glycol graft copolymer, wherein the ratio by weight of Aprepitant to the carrier polyvinyl caprolactam - polyvinyl acetate - polyethylene glycol graft copolymer is from 1:1 to 1:4, which pharmaceutical composition further comprises a diluent, a disintegrant, a glidants and a lubricant and wherein the diluent is lactose.

2. A process for the preparation of a pharmaceutical composition of Aprepitant comprising the steps of:
- Mixing the granules of an amorphous solid dispersion as defined in claim 1 with a diluent, a disintegrant and a glidant, wherein the diluent is lactose until complete homogeneity is achieved;
- Further mixing with a lubricant and filling hard gelatin capsules with the obtained granules,

## Patentansprüche

1. Pharmazeutische Zusammensetzung bestehend aus einer amorphen festen Dispersion von Aprepitant mit Polyvinylcaprolactam-Polyvinylacetat-Polyethylenglykol-Pfropfcopolymer, wobei das Gewichtsverhältnis von Aprepitant zu dem Träger Polyvinylcaprolactam-Polyvinylacetat-Polyethylenglykol-Pfropfcopolymer 1:1 bis 1:4 beträgt, **dadurch gekennzeichnet, dass** die pharmazeutische Zusammensetzung ferner ein Verdünnungsmittel, ein Sprengmittel, ein Gleitmittel und ein Schmiermittel umfasst, worin das Verdünnungsmittel Lactose ist.

2. Verfahren für die Zubereitung einer pharmazeutischen Zusammensetzung von Aprepitant, welches aus den nachstehenden Schritten besteht:
- Das Granulat einer amorphen festen Dispersion nach Anspruch 1 mit einem Verdünnungsmittel, einem Sprengmittel und einem Gleitmittel vermischen, worin das Verdünnungsmittel Lactose ist, bis eine vollständige Homogenität erzielt wird;
- Weiteres Mischen mit einem Gleitmittel und das erhaltene Granulat in Hartgelatinekapseln befüllen,

## Revendications

1. Composition pharmaceutique comprenant une dispersion solide amorphe d'Aprépitant avec un copolymère greffé par polyvinyle caprolactame - acétate de polyvinyle - polyéthylène glycol, dans laquelle le rapport en poids de l'Aprépitant au support copolymère greffé de polyvinyle caprolactame - acétate de polyvinyle - polyéthylène glycol est de 1:1 à 1:4; cette composition pharmaceutique comprend en outre un diluant, un désintégrant, un glissant et un lubrifiant et son diluant est le lactose.

2. Un procédé de préparation d'une composition pharmaceutique d'Aprépitant qui comprend les étapes suivantes:
- le mélange des granulés d'une dispersion solide amorphe telle que définie à la 1ère affirmation avec un diluant, un désintégrant et un glissant, où le diluant est le lactose jusqu'à l'homogénéité complète;
- le mélange ultérieur avec un lubrifiant et remplissage des capsules dures de gélatine par les granulés obtenus
